# EUROPÄISCHE PATENTSCHRIFT

(11) **EP 1 658 259 B1**
(45) Veröffentlichungstag und Bekanntmachung des Hinweises auf die Patenterteilung: **25.07.2012**
(21) Anmeldenummer: 04764111.3
(22) Anmeldetag: 13.08.2004
(51) Int. Cl.: C07C 67/307, C07C 69/34

(54) **VERFAHREN ZUR HERSTELLUNG VON a-FLUOR-MALONSÄUREDIALKYLESTERN**
METHOD FOR PRODUCING a-FLUOROMALONIC ACID DIALKYL ESTERS
PROCEDE DE PRODUCTION DE DIALKYLESTERS DE L'ACIDE a-FLUOROMALONIQUE

(30) Priorität: 18.08.2003 DE 10337885
(43) Veröffentlichungstag der Anmeldung: 24.05.2006
(60) Teilanmeldung: 10175679.9
(73) Patentinhaber: Bayer CropScience AG, 40789 Monheim (DE)
(72) Erfinder: GÜNTHER, Andreas, 51069 Köln (DE); WEINTRITT, Holger, 40764 Langenfeld (DE); BÖHM, Stefan, 51373 Leverkusen (DE)
(86) Internationale Anmeldenummer: PCT/EP2004/009117
(87) Internationale Veröffentlichungsnummer: WO 2005/019154

(56) Entgegenhaltungen:
- DE-A- 4 237 882
- DE-A- 10 041 618

## Beschreibung

Die Erfindung betrifft ein neues, vorteilhaftes Verfahren zur Herstellung von α-Fluormalonsäuredialkylestem.

α-Fluor-malonsäuredialkylester sind Zwischenprodukte, die beispielsweise für die Herstellung von 4,6-Dichlor-5-fluorpyrimidin (vgl. EP-A-0 970 057) verwendet werden. 4,6-Dichlor-5-fluorpyrimidin ist ein wichtiges Zwischenprodukt zur Herstellung von Wirkstoffen, die als Pflanzenschutzmittel eingesetzt werden (vgl. EP-A-0 882 043 und EP-A-0 937 050).

Es ist bereits bekannt geworden, dass man α-Fluor-β-ketoester der Formel (I) ausgehend von α-Chlor-β-ketoestern der Formel (II) durch Reaktion mit einem Anlagerungsprodukt von Fluorwasserstoff an ein Trialkylamin bei Temperaturen von 103 °C bis 130°C unter Druck erhalten kann (vgl. DE-A - 100 41 618).

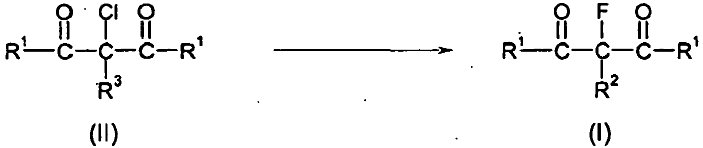

Ein wesentlicher Nachteil dieses Verfahrens ist, dass das Arbeiten unter Druck einen erhöhten apparativen Aufwand und besondere sicherheitstechnische Maßnahmen erfordert. Aus diesem Grund ist dieses Verfahren für die großtechnische Anwendung ungeeignet.

In einem anderen Verfahren (vgl. DE-A-42 37 882) erfolgt die Darstellung von α-Fluor-β-dicarbonylverbindungen der Formel (B) ausgehend von Dicarbonylverbindungen der Formel (A) durch Reaktion mit einem Anlagerungsprodukt von Fluorwasserstoff an ein Trialkylamin bei Temperaturen von 20°C bis 100°C.

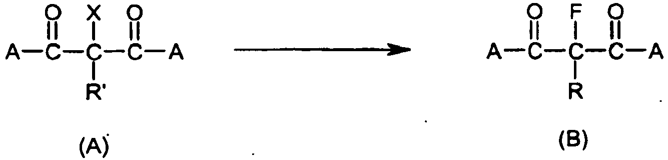

Nachteilig ist bei diesem Verfahren im Falle der Herstellung von α-Fluormalonsäuredialkylestern die lange Reaktionszeit, die trotz Anwendung hoher Überschüsse an Fluorwasserstoff und Triethylamin 72 Stunden beträgt.

Aufgabe der vorliegenden Erfindung war es nun, ein Verfahren zur Herstellung von α-Fluormalonsäuredialkylestern bereitzustellen, das die Darstellung ohne Anwendung von Überdruck in guten Ausbeuten und kürzeren Reaktionszeiten trotz niedriger Überschüsse an Fluorwasserstoff und Triethylamin ermöglicht, wodurch die Raum-Zeit-Ausbeute verbessert wird. Insbesondere sollte ein Verfahren gefunden werden, das durch den geringeren Verbrauch an Ausgangsverbindungen umweltfreundlicher ist.

Es wurde nun gefunden, dass man α-Fluormalonsäuredialkylester der allgemeinen Formel (I), 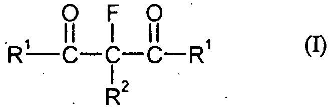 in welcher
- R¹: für Alkoxy mit 1 bis 4 Kohlenstoffatomen steht und
- R²: für Wasserstoff oder Fluor steht,
erhält, wenn man eine Dicarbonylverbindung der allgemeinen Formel (II), 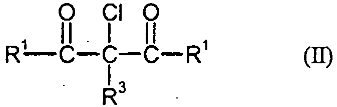 in welcher
- R¹: die oben angegebene Bedeutung hat und
- R³: für Wasserstoff, Fluor oder Chlor steht,
mit 1,4 bis 2 Mol - Äquivalenten eines angelerungsprodukts von Fluorwasserstoff an Triethylamin bei Temperaturen von 103°C bis 115°C bei 800 bis 1200 mbar umsetzt.

In den Verbindungen der Formel (II) steht R¹ insbesondere für Methoxy oder Ethoxy.

In den Verbindungen der Formel (II) steht R¹ besonders bevorzugt für Ethoxy.

In den Verbindungen der Formel (I) steht R² insbesondere für Wasserstoff.

Die oben aufgeführten oder in Vorzugsbereichen angegebenen Restedefinitionen gelten sowohl für die Ausgangverbindungen der Formel (II) als auch entsprechend für die Endprodukte der Formel (I).

Es ist als ausgesprochen überraschend zu bezeichnen, dass beim erfindungsgemäßen Verfahren; das unter Normaldruck und nur mit geringen Überschüsssen an Fluorwasserstoff und Triethylamin durchgeführt wird, eine bis zu zehnfach bessere Raum-Zeit-Ausbeute erzielt werden kann. Besonders überraschend ist, dass α-Fluormalonsäuredialkylester in ebenso guten Ausbeuten erhalten werden wie bei den im Stand der Technik beschriebenen Verfahren, die bei Normaldruck und niedrigeren Temperaturen durchgeführt werden, da der Fachmann bei höheren Temperaturen eine partielle Zersetzung und folglich niedrigere Ausbeuten erwartet.

Das erfindungsgemäße Verfahren weist eine Reihe von Vorteilen auf. So werden α-Fluormalonsäuredialkylester schon nach einem Bruchteil der Reaktionszeit erhalten, die bei bekannten Verfahren unter Normaldruck üblich ist. Nach dem erfindungsgemäßen Verfahren beträgt die Reaktionszeit 15 Stunden, während bei bekannten Verfahren 72 Stunden Reaktionszeit erforderlich sind (vgl. DE-A 42 37 882). Von besonderer Bedeutung ist, dass eine gute Raum-Zeit-Ausbeute erzielt wird, obwohl die Reaktion bei Normaldruck durchgeführt werden kann. Ein weiterer Vorteil besteht darin, dass trotz der relativ kurzen Reaktionszeit nur geringe Überschüsse an Fluorwasserstoff und Triethylamin nötig sind. Daher ist das neue Verfahren insbesondere für die großtechnische Anwendung gut geeignet.

Die Dicarbonylverbindungen der allgemeinen Formel (II) und alle anderen Ausgangsverbindungen sind gängige Handelsprodukte oder können durch einfache Verfahren aus diesen hergestellt werden.

Zur Durchführung des erfindungsgemäßen Verfahrens werden im Allgemeinen Anlagerungsprodukte von Fluorwasserstoff an Triethylamin verwendet, die pro Mol Triethylamin 1 bis 2 Mole Fluorwasserstoff enthalten, vorzugsweise pro Mol Triethylamin 1,2 bis 1,8 Mole Fluorwasserstoff, besonders bevorzugt 1,4 bis 1,5 Mole.

Zur Durchführung des erfindungsgemäßen Verfahrens werden im Allgemeinen pro Mol Ausgangsverbindung der Formel (III) 1,4 bis 2 Mole Triethylamin als Anlagerungsprodukt mit Fluorwasserstoff verwendet.

Die Anlagerungsprodukte von Fluorwasserstoff an Triethylamin können *in situ* durch Zudosieren von Triethylamin zu flüssigem Fluorwasserstoff hergestellt werden. Alternativ können die Anlagerungsprodukte von Fluorwasserstoff an Triethylamin *in situ* durch Zudosieren von Fluorwasserstoff zu Triethylamin erhalten werden.

Die Reaktionstemperaturen liegen bei der Durchführung des erfindungsgemäßen Verfahrens in dem Bereich von 103°C bis 115°C, vorzugsweise bei Temperaturen von 105°C bis 110°C.

Das erfindungsgemäße Verfahren wird unter Normaldruck durchgeführt. Unter Normaldruck im Sinne der Erfindung werden Drücke von 800 bis 1200 mbar verstanden.

Zur Durchführung des erfindungsgemäßen Verfahrens geht man im Allgemeinen wie folgt vor: In einem Reaktionsbehälter wird das Anlagerungsprodukt von Fluorwasserstoff an Triethylamin vorgelegt. Die Dicarbonylverbindungen der allgemeinen Formel (II) werden sofort oder beim Erwärmen dazudosiert. Dann wird das Reaktionsgemisch auf 105°C bis 110°C erhitzt und nachgerührt. Anschließend wird abgekühlt und mit Wasser versetzt. Die organische Phase wird abgetrennt und gegebenenfalls destilliert. Zur besseren Trennung des Produktes vom Wasser kann der einmalige oder mehrmalige Einsatz eines Extraktionsmittels vorteilhaft sein. Beispielhaft können Xylol, Toluol oder Methylenchlorid verwendet werden.

Vorzugsweise wird bei der Reaktionstemperatur so lange nachgerührt, bis das Ausbeuteoptimum erreicht ist.

Das erfindungsgemäße Verfahren wird beispielsweise zur Herstellung von α-Fluormalonsäurediethylester verwendet, der beispielsweise als Zwischenprodukt für die Herstellung von 4,6-Dichlor-5-fluorpyrimidin (vgl. EP-A-970 057) verwendet werden kann. 4,6-Dichlor-5-fluorpyrimidin wird zur Herstellung von Fluor-substituierten Heterocyclen verwendet werden, die beispielsweise biologisch aktiv oder als Zwischenprodukte für Pflanzenschutzmittel von Interesse sind (vgl. N. Ishikawa, J. Fluorine Chem. 1984, 25, 203, oder EP-A 970 057).

Das nachfolgende Beispiel dient zur Erläuterung der Erfindung. Die Erfindung ist jedoch nicht auf das Beispiel limitiert.

### Herstellungsbeispiele

### Beispiel 1 α-Fluormalonsäurediethylester

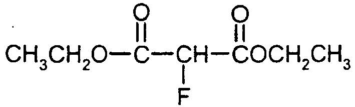

137 g (0,85 mol) Triethylamin-trishydrofluorid werden vorgelegt. 86 g (0,85 mol) Triethylamin werden bei 80°C zugegeben. Dann werden bei 80°C in 2 Stunden 195 g (1 mol) α-Chlormalonsäurediethylester zudosiert. Anschließend rührt man 15 Stunden bei Rückfluss (105 bis 110°C) unter Normaldruck nach. Zur Isolierung des Reaktionsproduktes gibt man bei 60°C 200 g Xylol und dann 215 g Wasser zur Reaktionsmischung und trennt dann die Phasen bei 60°C. Die wässrige Phase wird mit 100 g Xylol extrahiert.

Die beiden organischen Phasen werden vereinigt und im Vakuum destilliert. Die erste Fraktion ist Xylol. Die zweite Fraktion (156 g) enthält α-Fluormalonsäurediethylester mit 96 % Gehalt. Das sind 0,84 mol oder 84 % Ausbeute.

## Patentansprüche

1. Verfahren zur Herstellung von Verbindungen der allgemeinen Formel (I), 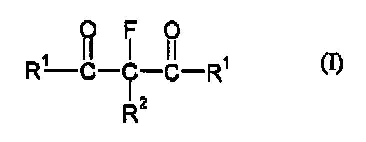 in welcher
R¹ für Alkoxy mit 1 bis 4 Kohlenstoffatomen steht und
R² für Wasserstoff oder Fluor steht,
**dadurch gekennzeichnet, dass** man eine Verbindung der allgemeinen Formel (II), 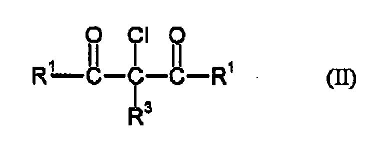 in welcher
R¹ die oben angegebene Bedeutung hat und
R³ für Wasserstoff, Fluor oder Chlor steht,
mit 1,4 bis 2 Mol-Äquivalenten eines Anlagerungsprodukts von Fluorwasserstoff an Triethylamin bei Temperaturen von 103°C bis 115°C bei 800 bis 1200 mbar umsetzt.

2. Verfahren gemäß Anspruch 1, **dadurch gekennzeichnet, dass** in den Verbindungen der Formeln (I) und (II) R¹ für Methoxy oder Ethoxy steht.

3. Verfahren gemäß mindestens einem der Ansprüche 1 bis 2, **dadurch gekennzeichnet, dass** in den Formeln (I) und (II) R² und R³ jeweils für Wasserstoff stehen.

4. Verfahren gemäß mindestens einem der Ansprüche 1 bis 3, **dadurch gekennzeichnet, dass** das Verfahren bei Temperaturen von 105°C bis 110°C durchgeführt wird.

5. Verfahren gemäß mindestens einem der Ansprüche 1 bis 4, **dadurch gekennzeichnet, dass** das Anlagerungsprodukt von Fluorwasserstoff an Triethylamin pro Mol Triethylamin 1,2 bis 1,8 Mole Fluorwasserstoff enthält.

## Claims

1. Process for praparing compounds of the general formula (I), 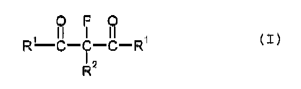 where
R¹ is alkoxy of 1 to 4 carbon atoms and
R² is hydrogen or fluorine,
**characterized in that** a compound of the general formula (II), 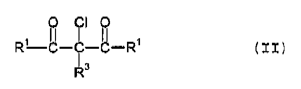 where
R¹ is as defined above and
R³ is hydrogen, fluorine or chlorine,
is reacted with 1.4 to 2 mol equivalents of an addition product of hydrogen fluorite with triethylamine at temperatures of 103°C to 115°C at 800 to 1200 mbar.

2. Process according to Claim 1, **characterized in that** R¹ is methoxy or ethoxy in the compounds of the formulae (I) and (II).

3. Process according to at least one of Claims 1 and 2, **characterized in that** R² and R³ are each hydrogen in the formulae (I) and (II).

4. Process according to at least one of Claims 1 to 3, **characterized in that** the profess is carried out at temperatures of 105°C to 110°C.

5. Process according to at least one of Claims 1 to 4, **characterized in that** the addition product of hydrogen fluoride with triethylamine contains 1.2 to 1.8 mol of hydrogen fluoride per mole of triethylamine.

## Revendications

1. Procédé pour la préparation de composés de formule générale (I), 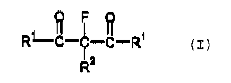 dans laquelle
R¹ représente un groupe alcoxy ayant de 1 à 4 atomes de carbone et
R² représente une atome d'hydrogène ou de Fluor,
**caractérisé en ce qu'**on fait réagir un composé de formule générale (II), 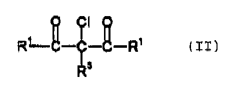 dans laquelle
R¹ a la signification indiquée ci-dessus et
R³ représentant un atome d'hydrogène, de fluor ou de chlore,
avec 1,4 à 2 équivalents molaires d'un produit de fixation par addiction d'acide fluorhydrique sur la triéthylamine à des températures de 103 °C à 115 °C sous 800 à 1 200 mbars.

2. Procédé selon la revendication 1, **caractérisé en ce que** dans les composés de formules (I) et (II) R¹ représente le groupe méthoxy ou éthoxy.

3. Procédé selon au moins l'une des revendications 1 et 2, **caractérisé en ce que** dans les formules (I) et (II) R² et R³ représentent chacun un atome d'hydrogène.

4. Procédé selon au moins l'une des revendications 1 à 3, **caractérisé en ce que** le procédé est effectué à des températures de 105 °C à 110 °C.

5. Procédé selon au moins l'une des revendications 1 à 4, **caractérisé en ce que** le produit de fixation par addition d'acide fluorhydrique sur la triéthylamine contient de 1,2 à 1,8 moles d'acide fluorhydrique par mole de triéthylamine,
